# EUROPEAN PATENT APPLICATION

(11) **EP 4 599 676 A1**
(43) Date of publication of application: **13.08.2025**
(21) Application number: 24157157.9
(22) Date of filing: 12.02.2024
(51) Int. Cl.: A01N 59/00, B08B 3/00, A01P 1/00, A01N 25/06, A01N 25/16, A01N 25/22, A01N 37/16

(54) **A CONTAINER**

(71) Applicant: Aspire Technology Group Ltd, St Albans Hertfordshire AL1 3RD (GB)
(72) Inventor: The designation of the inventor has not yet been filed
(74) Representative: Stepney, Gregory John

(57) **Abstract**

A container containing an aqueous composition comprising one or more peroxides is provided. The container includes an internal surface which is coated with a polyamideimide (PAM) coating.

## Description

### FIELD OF THE INVENTION

This invention relates to a container in which an aqueous composition comprising one or more peroxides is retained.

### BACKGROUND

Peroxides are effective broad-spectrum biocides which are used to disinfect surfaces, materials and equipment contaminated by bacteria, fungi, viruses, amoebae and other pathogens. In some cases, they are also capable of removing biofilms. Hydrogen peroxide in particular is a desirable disinfectant because it forms no harmful by-products, is pH neutral when dosed, and is odourless and colourless. After disinfection, it degrades into water and oxygen. Peroxides are relatively unstable due to the presence of the oxygen-oxygen bond which makes them prone to decomposition and affects their shelf life and longevity. Factors such as high concentration, elevated temperature and exposure to light can accelerate decomposition and have to be controlled in order to prolong the storage life of peroxide products.

Peroxide solutions are stored in unpressurised containers due to the unavoidable evolution of oxygen that occurs during decomposition. This can potentially lead to rupture of the container in which the solution is held or combustion of the evolved oxygen gas, both of which pose a hazard. Despite this risk, it may be advantageous to be use pressurised solutions of peroxide disinfectants, for example, when disinfecting large spaces by fumigation using a peroxide solution. Therefore, there is a need to provide a means of storing a pressurised peroxide solution which alleviates the aforementioned degradation problem at least to some extent.

### SUMMARY OF THE INVENTION

In accordance with a first aspect of the invention, there is provided a container containing an aqueous composition comprising one or more peroxides, wherein an internal surface of the container is coated with a polyamide-imide (PAM) coating.

Advantageously, the PAM coating provides a stabilising effect on the peroxides which permits the composition to be stored for extended periods of time without appreciable degradation of the one or more peroxides. The PAM coating provides a barrier between the container wall and composition which, in embodiments in which the container comprises a metal, avoids metal-catalysed decomposition of the peroxide.

The internal pressure of the container may be higher than the external, ambient pressure. The container may be suitable for expelling the composition as an aerosol. The stabilising effect of the PAM coating may permit the peroxide composition to be stored at elevated pressures without a risk of decomposition of the peroxide and the consequent evolution of oxygen gas. This may allow the composition to be pressurised and used as an aerosol with minimal risk that the container will rupture during transport or storage.

The one or more peroxides may comprise at least one of hydrogen peroxide, peracetic acid, performic acid, peroxymonosulfuric acid, peroxynitric acid, and peroxymonophosphoric acid. For example, the one or more peroxides may comprise at least one of hydrogen peroxide and peracetic acid, or a mixture of hydrogen peroxide and peracetic acid. These peroxides represent the most effective peroxide sterilants which may all advantageously be stabilised in the container.

The peroxide may be present in the composition in an amount of 0.01 wt% or higher, from 0.01 to 4.5 wt% or 0.1 to 4.5 wt%, or 0.5 to 4.0 wt%, or 1.0 to 3.0 wt%. This range represents the most effective and safe peroxide concentration range for a ready-to-use sterilant.

The container may comprise aluminium or an aluminium alloy. Aluminium is a preferred material for the container due to its low weight, structural rigidity and recyclability. Although aluminium can react with peroxide, the PAM coating provides a barrier which prevents contact between the aluminium and peroxide.

The container may be impervious to light. By preventing light from entering the interior of the container, UV-induced degradation of the peroxide can be reduced and the shelf life of the composition extended.

The container may contain a propellant. Preferably the propellant may be selected from one or more of nitrogen, dimethyl ether (DME), propane, n-butane, and iso-butane. The propellant may permit the composition to be aerosolised, which may enable its use in applications in which fumigation of a large area is required.

The container may be formed by extrusion of a billet of aluminium or aluminium alloy. The use of extrusion to form the container advantageously provides a smooth inner surface on the container walls and avoids the presence of imperfections or mould seams which can result from other manufacturing processes. This minimises the likelihood of perforations in the PAM coating forming, which would otherwise allow the peroxide to come into contact with the aluminium of the container wall and decompose.

The aluminium alloy may comprise one or more of copper, magnesium, silicon, manganese, tin and zinc. The use of these metals in the alloy may provide advantageous properties such as enhanced strength, good weldability and/or corrosion resistance.

The PAM coating may have a thickness of from 0.1 µm to 5 µm. This range may provide the optimal thickness required to fully coat the interior of the container while minimising production costs.

The PAM may have a number average molecular weight of from 1000 to 60,000, such as from 5,000 to 30,000, from 10,000 to 20,000, or about 15,000. PAM polymers within these ranges may provide an optimal stabilising effect on the peroxide.

The coating may be applied by spray coating and/or spin spray coating. These methods may advantageously be suitable for providing a complete coating of the container inner wall and/or provide a uniform coating thickness.

In accordance with a second aspect of this invention, there is provided a use of a container containing an aqueous composition comprising one or more peroxides for sterilising a substrate, wherein an internal surface of the container is coated with a polyamide-imide (PAM) coating.

The sterilising may comprise environmental disinfection of the substrate. Advantageously, the aqueous composition comprising one or more peroxides may be suitable for disinfecting a large area such that the environment of the area is entirely or almost entirely disinfected.

The sterilizing may comprise spraying the aqueous composition on the substrate. The spraying may comprise forming an aerosol of the composition, for example under elevated pressure. The sterilising may comprise sterilising the surface to remove spores, such as bacterial spores. The efficacy of the composition is preferably such that it has biocidal activity against difficult to kill microbes and biofilms despite being stored in the container for extended periods of time.

The container may be sealed with a valve. The valve may be a one-shot (pulse) valve. This may permit the composition to be expelled from the container in a selective manner. In other embodiments, an alternative valve may be provided to permit the entire contents of the container to be ejected in a single activation. This may have utility in fumigation applications.

In accordance with a third aspect of this invention, there is provided a use of a polyamide-imide (PAM) coated container to stably store an aqueous composition comprising one or more peroxides.

In accordance with a fourth aspect of this invention, there is provided a method for stabilising an aqueous composition comprising one or more peroxides, the method comprising storing the composition in a container comprising a polyamide-imide (PAM) coating.

The advantages associated with the first aspect apply equally to the second, third and fourth aspects.

Unless otherwise stated, each of the integers described may be used in combination with any other integer as would be understood by the person skilled in the art. Further, although all aspects of the invention preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

As used herein and in the accompanying claims, unless the context requires otherwise, "comprise" or variations such as "comprises" or "comprising" will be understood to imply the inclusion of a stated integer or group of integers but not the exclusion of any other integer or group of integers.

The term "consist(s)/(ing) essentially of", with respect to the components of a composition, alloy or mixture, means the composition, alloy or mixture contains the indicated components and may contain minor additional components in an amount less than 1 wt% based on the total weight of the composition, alloy or mixture, and provided that the additional components do not substantially alter the reactivity of the composition, alloy or mixture.

Further, in the discussion of the invention, unless stated to the contrary, the disclosure of alternative values for the upper or lower limit of the permitted range of a parameter is to be construed as an implied statement that each intermediate value of the said parameter, lying between the smaller and greater of the alternatives, is itself also disclosed as a possible value for the parameter.

In addition, unless otherwise stated, all numerical values appearing in this application are to be understood as being modified by the term "about". As used herein, the term "about" means that the stated value can vary by ± 10%. For example, about 90 wt% means 90±9 wt%, and about 0.1 wt% means 0.1±0.01 wt%. When used with reference to a range, the term "about" applies to all values in the range.

In order that the invention may be more readily understood, it will be described further with reference to the figures and to the specific examples hereinafter.

### DETAILED DESCRIPTION OF THE INVENTION

The present disclosure provides a container containing an aqueous composition comprising one or more peroxides, wherein an internal surface of the container is coated with a polyamide-imide (PAM) coating.

The container may be configured to form and expel the composition as an aerosol. To this end, in some embodiments the internal pressure of the container may be higher than the external, ambient pressure. For example, the internal pressure of the container may be from about 1200 kPa (absolute) to about 500 kPa (absolute) , from 1500 kPa (absolute) to about 400 kPa (absolute), or from about 200 kPa (absolute) to about 3 kPa (absolute). The container may contain a propellant for aerosolising the composition. The propellant may be selected from one or more of nitrogen, dimethyl ether (DME), propane, n-butane, and iso-butane. In some embodiments, the propellant may be dimethyl ether (DME). The propellant may be present in the composition in an amount of from 1 wt% to 99 wt%, from 5 wt% to 80 wt%, from 10 wt% to 60 wt%, from 15 wt% to 50 wt%, from 20 wt% to 40 wt%, from 20 wt% to 30 wt%, or about 25 wt%. Preferably the propellant is present in an amount of from 20 wt% to 40 wt%, or about 25 wt%.

The container may comprise aluminium or an aluminium alloy. The aluminium alloy may comprise one or more of copper, magnesium, silicon, manganese, tin and zinc. The use of these metals in the alloy may provide advantageous properties such as enhanced strength, good weldability and/or corrosion resistance. The container may be formed by extrusion of a billet of aluminium or aluminium alloy. This process avoids the formation of seams or point imperfections in the internal surface of the container, which may perforate the PAM coating and expose the peroxide to the container wall.

The container may be impervious to light, particularly in embodiments in which the container comprises aluminium or aluminium alloy. It is important to ensure that UV radiation is excluded from the interior of the container to avoid degradation of the peroxide. In some embodiments, for example where a wall of the container is light permeable, a UV impermeable coating may be applied to the wall.

In order to permit the composition to exit the container, a mouth of the container may be sealed with a valve. The valve may be a one-shot (pulse) valve. Pulse valves provide a momentary (pulsed) output at the container exit port when pressure is applied at the inlet. No additional flow is possible until pressure at the inlet is removed, reset time allowed, and pressure reapplied. This may allow selective ejection of the composition from the container. In other embodiments, a valve may be provided which permits the entire contents of the container to be ejected in a single activation. This may be of particular use in fumigation applications. The valve may be secured to the container by methods commonly known in the art.

The one or more peroxides may comprise at least one of hydrogen peroxide, peracetic acid, performic acid, peroxymonosulfuric acid, peroxynitric acid, and peroxymonophosphoric acid. For example, the one or more peroxides may comprise at least one of hydrogen peroxide and peracetic acid, or a mixture of hydrogen peroxide and peracetic acid. These peroxides represent the most effective peroxide sterilants and they may all be advantageously stabilised in the container.

The peroxide may be present in the composition in an amount of 0.01 wt% or higher, from 0.01 to 4.5 wt%. In some embodiments, the peroxide may be present in the composition in an amount of from 0.1 to 4.5 wt%, from 0.1 to 3.0 wt%, from 0.2 to 2.5 wt%, or about 1.5 wt%.

The composition may further comprise one or more adjuvants such as water, an organic acid (e.g. formic acid, acetic acid, or propionic acid), or a solvent (e.g. acetonitrile, methanol, ethanol, propanol, isopropanol, butanol or isobutanol).

The polyamide-imide (PAM) coating may provide a strong, thermally stable, chemically resistant barrier on the internal wall of the container which may serve to stabilise the peroxide. Furthermore, the polyamide-imide coating is resistant to deformation or creep due to the strong intermolecular interactions arising from the polyimide functional groups and the ability of the polymer chains to hydrogen bond with one another through the amide bond. Advantageously, the PAM coating may provide a stabilising effect which permits the composition to be stored for extended periods of time without appreciable degradation of the one or more peroxides. The PAM may coat the entire internal surface of the container. In some embodiments, the PAM coating may comprise gold pam-2-c-internal liner 8460 N. An example of a PAM coating which is suitable for use in the present invention is described in WO2014096075A1, the contents of which is incorporated herein by reference.

The PAM coating may have a thickness of from 0.1 µm to 5 µm, from 0.5 µm to 4 µm, from 1 µm to 3 µm, from 1.5 µm to 2.5 µm or about 2 µm. This range may provide the optimal thickness required to fully coat the interior of the container while minimising production costs.

The PAM may have a number average molecular weight of from 1000 to 60,000, from 5,000 to 30,000, from 10,000 to 20,000, or about 15,000. PAM polymers within these ranges may provide an optimal stabilising effect on the peroxide.

The coating may be applied by spray coating and/or spin spray coating to provide a uniform covering of the internal container wall. However, any alternative coating method which provides a uniform coating may alternatively be used.

The composition may be capable of providing environmental disinfection of a substrate to which it is applied. The sterilizing may comprise spraying the aqueous composition on the substrate. The substrate may include small discrete surfaces of workspaces, tools and machinery, or alternatively, large areas such as hospital rooms, laboratories, or workshops. Where disinfection of a large area is required, the composition may be aerosolised, ejected into the air, and allowed to settle on the surface. This may result in 'environmental disinfection' in which the area is entirely or almost entirely disinfected.

The invention will now be described in further detail regarding the following non-limiting examples.

### EXAMPLES

### Example 1:

A mixing vessel was charged with deionised water to a specific, chosen w/w value. All further ingredients, optionally including an adjuvant, was added sequentially to a proprietary formula to specific w/w values, mechanically stirring at each stage. A lab sample was collected for final verification of H₂O₂ and Peracetic acid (PAA) content.

### Example 2:

An aluminum / aluminum alloy puck was placed on an automatic mandrel. An extrusion process initiated with a hydraulic ram and forming tool made contact with the pucks outer surface and stretched it over a preformed shaped mould to form an aluminum flask suitable for storing a composition of the invention. A polyamide-imide (PAM) coating was then applied to the inside of the aluminum flask by spin-coating, in accordance with the detail description section of this application.

### Example 3:

Samples of a composition comprising about 74 wt% water, 25 wt% DME, 0.22 wt% hydrogen peroxide, 0.05 wt% peracetic acid, and about 0.1 wt% acetic acid were prepared according to Example 1 and stored in aluminum flasks having an internal PAM coating prepared according to Example 2. The flasks were sealed with a pulse valve and stored at temperatures ranging from 5 °C to 50 °C for periods of up to 7 years. Thereafter in an annual cycle the compositions were sprayed onto petri dishes inoculated with bacterial spores and, after a period of 10 minutes, swabs of the surfaces were taken and tested. In all cases, no detectable contamination of the surfaces was measurable. These tests show that the peroxide had not degraded despite several years of storage.

Chemical concentration tests were also conducted and recorded showing no deterioration of the peroxide.

The results of the swab tests and the chemical concentrations tests are shown in the table below. The tests were performed on an annual cycle after 7 years of storage as described above. Each test was performed on 4 samples.

| **Sample** | **Test** | **Year 8** | **Year 9** | **Year 10** | **Year 11** | **Year 12** | **Year 13** | **Year 14** |
|---|---|---|---|---|---|---|---|---|
| 1 | Spores detected | 0 | 0 | 0 | 0 | 0 | 0 | 0 |
| | Peroxide concentration (wt%) | > 0.28 | > 0.28 | > 0.28 | > 0.28 | > 0.28 | > 0.28 | > 0.28 |
| | | | | | | | | |
| 2 | Spores detected | 0 | 0 | 0 | 0 | 0 | 0 | 0 |
| | Peroxide concentration (wt%) | > 0.28 | > 0.28 | > 0.28 | > 0.28 | > 0.28 | > 0.28 | > 0.28 |
| | | | | | | | | |
| 3 | Spores detected | 0 | 0 | 0 | 0 | 0 | 0 | 0 |
| | Peroxide concentration (wt%) | > 0.28 | > 0.28 | > 0.28 | > 0.28 | > 0.28 | > 0.28 | > 0.28 |
| | | | | | | | | |
| 4 | Spores detected | 0 | 0 | 0 | 0 | 0 | 0 | 0 |
| | Peroxide concentration (wt%) | > 0.28 | > 0.28 | > 0.28 | > 0.28 | > 0.28 | > 0.28 | > 0.28 |

As shown in the table, there was no degradation of peroxide over time and no decrease in the efficacy of the disinfectant as evidenced by the absence of spores. These results demonstrate that the composition of the invention is stable over a long period of time.

It would be appreciated that the process and apparatus of the invention are capable of being implemented in a variety of ways, only a few of which have been illustrated and described above.

The present invention can be further understood by reference to the following clauses:
1. A container containing an aqueous composition comprising one or more peroxides, wherein an internal surface of the container is coated with a polyamide-imide (PAM) coating.
2. The container according to clause 1, wherein the internal pressure of the container is higher than the external, ambient pressure.
3. The container according to any preceding clause, wherein the container is suitable for expelling the composition as an aerosol.
4. The container according to any preceding clause, wherein the one or more peroxides comprises at least one of hydrogen peroxide, peracetic acid, performic acid, peroxymonosulfuric acid, peroxynitric acid, and peroxymonophosphoric acid, such as at least one of hydrogen peroxide and peracetic acid, or a mixture of hydrogen peroxide and peracetic acid.
5. The container according to any preceding clause, wherein the peroxide is present in the composition in an amount of from 0.01 to 4.5 wt% or 0.1 to 4.5 wt%, or 0.5 to 4.0 wt%, or 1.0 to 3.0 wt%.
6. The container according to any preceding clause, wherein a mixture of hydrogen peroxide and peracetic acid is present in the composition in an amount of from 0.01 to 4.5 wt% or 0.1 to 4.5 wt%, or 0.5 to 4.0 wt%, or 1.0 to 3.0 wt%.
7. The container according to any of clauses 1 to 4, wherein hydrogen peroxide is present in the composition in an amount of from 0.01 to 4.5 wt% or 0.1 to 4.5 wt%, or 0.5 to 4.0 wt%, or 1.0 to 3.0 wt%.
8. The container according to any of clauses 1 to 4, wherein peracetic acid is present in the composition in an amount of from 0.01 to 4.5 wt% or 0.1 to 4.5 wt%, or 0.5 to 4.0 wt%, or 1.0 to 3.0 wt%.
9. The container according to any preceding clause, wherein the container comprises aluminium or an aluminium alloy.
10. The container according to any preceding clause, wherein the container is impervious to light.
11. The container according to any preceding clause, wherein the container contains a propellant, preferably wherein the propellant is selected from one or more of nitrogen, dimethyl ether (DME), propane, n-butane, and iso-butane.
12. The container according to any preceding clause, wherein the container has been formed by extrusion of a billet of aluminium or aluminium alloy.
13. The container according to clause 12, wherein the aluminium alloy comprises one or more of copper, magnesium, silicon, manganese, tin and zinc.
14. The container according to any preceding clause, wherein the PAM coating has a thickness of from 0.1 µm to 5 µm.
15. The container according to any preceding clause, wherein the PAM has a number average molecular weight of from 1000 to 60,000, such as from 5,000 to 30,000, from 10,000 to 20,000, or about 15,000.
16. The container according to any preceding clause, wherein the coating is applied by spray coating and/or spin spray coating.
17. The container according to any preceding clause, wherein the PAM coating has a thickness of from 0.1 µm to 5 µm and the a foam-forming aqueous composition comprises a mixture of hydrogen peroxide and peracetic acid in an amount of from 0.01 to 4.5 wt%, hydrogen peroxide in an amount of from 0.01 to 4.5 wt% or peracetic acid in an amount of from 0.01 to 4.5 wt%, optionally wherein the foam is formulated to be deposited in a water pipe or drain.
18. The container according to any preceding claim, wherein the polyamide-imide (PAM) coating covers at least 95%, at least 99% or the whole of the internal surface forming the container.
19. The container according to any preceding clause, wherein the container is sealed with a valve, preferably wherein the valve is a one-shot (pulse) valve.
20. The container according to clause 19, wherein valve surfaces facing into the container do not contain a metal.
21. Use of a container containing an aqueous composition comprising one or more peroxides for sterilising a substrate, wherein an internal surface of the container is coated with a polyamide-imide (PAM) coating, optionally wherein the container is as defined in any of clauses 2 to 20.
22. The use according to clause 21, wherein the sterilising comprises environmental disinfection of the substrate.
23. The use according to clause 21 or clause 22, wherein the sterilizing comprises spraying the aqueous composition on the substrate.
24. The use according to any of clause 21 to 23, wherein the sterilising comprises sterilising spores.
25. The use according to any of clause 21 to 24, wherein the container is sealed with a valve, preferably wherein the valve is a one-shot (pulse) valve.
26. Use of a polyamide-imide (PAM) coated container to stably store an aqueous composition comprising one or more peroxides, optionally wherein the container is as defined in any of clauses 2 to 20.
27. A method for stabilising an aqueous composition comprising one or more peroxides, the method comprising storing the composition in a container comprising a polyamide-imide (PAM) coating, optionally wherein the container is as defined in any of clauses 2 to 20.

## Claims

1. A container containing an aqueous composition comprising one or more peroxides, wherein an internal surface of the container is coated with a polyamide-imide (PAM) coating, optionally wherein the peroxide is present in the composition in an amount of from 0.01 to 4.5 wt%.

2. The container according to claim 1, wherein the internal pressure of the container is higher than the external, ambient pressure.

3. The container according to any preceding claim, wherein the container is suitable for expelling the composition as an aerosol.
3.The container according to any preceding claim, wherein the one or more peroxides comprises at least one of hydrogen peroxide, peracetic acid, performic acid, peroxymonosulfuric acid, peroxynitric acid, and peroxymonophosphoric acid, such as at least one of hydrogen peroxide and peracetic acid, or a mixture of hydrogen peroxide and peracetic acid.

4. The container according to any preceding claim, wherein the container comprises aluminium or an aluminium alloy and/or wherein the container is impervious to light.

5. The container according to any preceding claim, wherein the container contains a propellant, optionally wherein the propellant is selected from one or more of nitrogen, dimethyl ether (DME), propane, n-butane, and iso-butane.

6. The container according to any preceding claim, wherein the container has been formed by extrusion of a billet of aluminium or aluminium alloy, optionally wherein the aluminium alloy comprises one or more of copper, magnesium, silicon, manganese, tin and zinc.

7. The container according to any preceding claim, wherein the PAM coating has a thickness of from 0.1 µm to 5 µm.

8. The container according to any preceding claim, wherein the PAM has a number average molecular weight of from 1000 to 60,000, such as from 5,000 to 30,000, from 10,000 to 20,000, or about 15,000.

9. The container according to any preceding claim, wherein the coating is applied by spray coating and/or spin spray coating.

10. Use of a container containing an aqueous composition comprising one or more peroxides for sterilising a substrate, wherein an internal surface of the container is coated with a polyamide-imide (PAM) coating, optionally wherein the sterilising comprises environmental disinfection of the substrate, optionally wherein the container is as defined in any of claims 1 to 9.

11. The use according to claim 10, wherein the sterilizing comprises spraying the aqueous composition on the substrate.

12. The use according to claim 10 or claim 11, wherein the sterilising comprises sterilising spores.

13. The use according to any of claims 10 to 12, wherein the container is sealed with a valve, optionally wherein the valve is a one-shot (pulse) valve.

14. Use of a polyamide-imide (PAM) coated container to stably store an aqueous composition comprising one or more peroxides, optionally wherein the container is as defined in any of claims 1 to 9.

15. A method for stabilising an aqueous composition comprising one or more peroxides, the method comprising storing the composition in a container comprising a polyamide-imide (PAM) coating, optionally wherein the container is as defined in any of claims 1 to 9.
